# EUROPÄISCHE PATENTSCHRIFT

(11) **EP 2 332 610 B1**
(45) Veröffentlichungstag und Bekanntmachung des Hinweises auf die Patenterteilung: **13.02.2013**
(21) Anmeldenummer: 09015292.7
(22) Anmeldetag: 10.12.2009
(51) Int. Cl.: A61M 39/10, A61J 1/14

(54) **Anschlusssystem für Nahrungsbehälter zur enteralen Ernährung**
Connection system for food containers for enteral feeding
Système de raccordement pour récipients d'aliments destinés à la nutrition entérale

(43) Veröffentlichungstag der Anmeldung: 15.06.2011
(73) Patentinhaber: Fresenius Kabi Deutschland GmbH, 61352 Bad Homburg v.d.H. (DE)
(72) Erfinder: Becker, Michael, Dr., 75438 Knittlingen (DE); Breuer-Thal, Barbara, 65510 Idstein (DE)
(74) Vertreter: Fresenius Kabi Deutschland GmbH

(56) Entgegenhaltungen:
- EP-A1- 1 837 005
- WO-A1-2008/028582
- WO-A1-2008/049568
- DE-C1- 10 146 007

## Beschreibung

### Technisches Gebiet

Die vorliegende Erfindung liegt auf dem Gebiet der enteralen Ernährung. Insbesondere wird ein Aufsatz für eine Vorrichtung zur enteralen Ernährung offenbart. Weiterhin betrifft die Erfindung eine Vorrichtung für die enterale Ernährung mit einem derartigen Aufsatz.

### Stand der Technik

Bei der enteralen Ernährung wird dem Patienten eine flüssige Nährlösung mit einer enteralen Ernährungssonde appliziert. Die enterale Nährlösung wird in einem Nahrungsbehälter bereitgestellt, der über eine sogenannte Überleiteinheit mit einer enteralen Ernährungssonde verbunden wird. Die Überleiteinheit weist einen Sondenschlauch mit einem distalen und proximalen Verbindungsstück auf. Das distale Verbindungsstück wird mit der Ernährungssonde und das proximale Verbindungsstück mit dem Nahrungsbehälter verbunden, so dass die Nährlösung aus dem Nahrungsbehälter über den Verbindungsschlauch der Überleiteinheit in die Ernährungssonde fließt.

Der Nahrungsbehälter wird in der Regel mehrmals täglich ausgetauscht, während bei der Überleiteinheit ein Wechsel alle 24 Stunden empfohlen wird. Die Ernährungssonde kann bis zu mehreren Monaten verwendet werden.

Zur Verbindung der Überleiteinheit mit der Ernährungssonde finden im Stand der Technik die bekannten Luer-Lock-Konnektoren Verwendung, sodass Überleiteinheit und Ernährungssonde miteinander verbunden werden können, ohne dass die Gefahr besteht, dass sich der Sondenschlauch der Ernährungssonde von dem Verbindungsschlauch der Überleiteinheit unbeabsichtigt löst. Alternativ werden auch sogenannte Stufenadapter eingesetzt, die distal mit Überleiteinheiten verbunden sind, und die mit semirigiden Trichteransätzen am proximalen Ende der Ernährungsonden über eine Press-fit-Verbindung konnektiert werden. Ein verbessertes Verbindungssystem zwischen Überleiteinheit und Sondenschlauch wurde in WO 2008/049568 A beschrieben.

Die Art der Verbindung des Nahrungsbehälters mit der Überleiteinheit ist unter anderem abhängig vom verwendeten Nahrungsbehälter, da die verschiedenen Hersteller solcher Nahrungsbehälter unterschiedliche technische Lösungen anbieten. In vielen Fällen wird zur Verbindung ein sogenannter Spike, d.h. Dorn bzw. Kanüle, verwendet, der mit dem Entnahmeteil (Port) des Nahrungsbehälters verbunden wird. Der Port ist hierbei als ein Verbindungsstutzen ausgebildet und dient sowohl als Kanal für den Spike, als auch als Befestigungsmittel für die Überleiteinheit. Der Spike penetriert dabei die Versiegelung des Nahrungsbehälters oder er penetriert eine Membran, die sich wieder verschließt, sobald der Spike entfernt wird. Beispielsweise wird bei den Nahrungsbehältern des Herstellers Nestle der Verbindungsstutzen direkt auf den komplett verschlossenen Nahrungsbehälter aufgebracht, während er bei den Herstellern Danone und Fresenius Kabi in die Folie des Nahrungsbehälters eingeschweißt wird. Bei letzteren beiden Herstellern wird daher zusätzlich eine versiegelnde Membran bzw. eine versiegelnde Abreißkappe auf das vom Nahrungsbehälter wegweisende Ende des Verbindungsstutzens aufgebracht, um die im Behälter enthaltene Nahrung keimfrei zu halten.

Nachteilig ist jedoch, dass bei diesen Verbindungsmöglichkeiten zwischen Nahrungsbehälter und Überleiteinheit ein Spike benötigt wird, um entweder die Nahrungsbehälterwand oder eine am Verbindungsstutzen angebrachte Versieglung zu durchstoßen. Dies birgt insofern ein erhebliches Risiko für den Patienten, da auch andere Verbindungssysteme, die insbesondere in der intravenösen Applikation eingesetzt werden, einen Spike aufweisen und somit prinzipiell zur Verbindung mit dem Nahrungsbehälter verwendet werden können, sofern diese dann über Adaptern mit enteralen Ernährungssonden verbunden werden. Wenn beispielsweise eine enterale Ernährungslösung irrtümlich über einen intravenös gelegten Zugang appliziert werden sollte, besteht Lebensgefahr für den Patienten.

WO 2008/028582 offenbart die Merkmale des Oberbegriffs des Anspruchs 1.

### Kurzbeschreibung der Erfindung

Die der Erfindung zugrundeliegende Aufgabe besteht in der Bereitstellung eines universell einsetzbaren, sicheren und gleichzeitig einfach zu handhabenden Anschlusssystems für Nahrungsbehälter zur enteralen Ernährung, sowie einer Vorrichtung für die enterale Ernährung mit einem solchen Anschlusssystem.

Die Lösung dieser Aufgaben erfolgt erfindungsgemäß mit den im Patentanspruch 1 angegebenen Merkmalen. Vorteilhafte Ausführungsformen werden in den Unteransprüchen, der Beschreibung und den Zeichnungen angegeben.

Der Aufsatz einer erfindungsgemäßen Vorrichtung zur enteralen Ernährung, umfasst zumindest zwei Verbindungsstücke, wobei das erste Verbindungsstück mit dem Verbindungsstutzen eines Nahrungsbehälters verbunden ist und das zweite Verbindungsstück mit einer Überleiteinheit verbunden ist, sodass ein Nahrungsfluss von Nahrungsbehälter durch Verbindungsstutzen und Aufsatz zur Überleiteinheit ermöglicht wird, wobei der Aufsatz ein Mittel zum Öffnen des Nahrungsbehälters umfasst.

Gemäß der vorliegenden Erfindung besitzt

der proximale Konnektor der Überleiteinheit keinen Spike mehr , bzw. ein einen Spike besitzender Konnektor ist inkompatibel zu einem Nahrungsbehälter, der mit einem solchen Aufsatz ausgerüstet ist.

Der proximale Konnektor der Überleiteinheit kann mit dem Aufsatz verbunden werden und bildet eine zur Umgebung hin geschlossene Einheit, die vor Kontamination der Nahrung schützt.

Der proximale Konnektor der Überleiteinheit kann mit dem Aufsatz des Nahrungsbehälters verbunden werden, bevor die Versiegelung des Nahrungsbehälters geöffnet wird, um das Kontaminationsriskio und das Leckagerisko zu minimieren.

Im Aufsatz ist das zumindest eine, bevorzugt mechanische, Mittel zum Öffnen der Versiegelung des Nahrungsbehälters integriert,

Der Aufsatz ist so ausgestaltet, dass ein für intravenöse Anwendungen ausgelegter Spike nicht geeignet ist, das Nahrungsbehältnis und seine Versiegelung durch den Aufsatz zu öffnen.

Dies wird erfindungsgemäß dadurch ermöglicht, dass der Aufsatz als Bindeglied zwischen Überleiteinheit und Nahrungsbehälter dient und zum einen mit sowohl Verbindungsstutzen des Nahrungsbehälters als auch der Überleiteinheit verbindbar ist, und zum anderen ein Mittel zur Öffnung des Nahrungsbehälters von dem Aufsatz umfasst wird. Somit wird es durch die vorliegende Erfindung vorteilhaft ermöglicht, einen Nahrungsbehälter an eine Überleiteinheit anzuschließen, ohne einen mit dem Schlauch der Überleiteinheit verbundenen Spike einsetzen zu müssen. Infolgedessen wird durch die vorliegende Erfindung ausgeschlossen, dass andere im klinischen Umfeld verwendete Verbindungstypen - beispielsweise bei intravenösen Applikation -, die einen Spike umfassen, mit dem Nahrungsbehälter verbunden werden können. Somit wird ein erheblicher Sicherheitsgewinn für den Patienten erzielt.

Die Vorrichtung zur enteralen Ernährung umfasst neben dem Aufsatz zumindest einen Nahrungsbehälter, an welchen ein Verbindungsstutzen angebracht ist, sowie eine Überleiteinheit und vorzugsweise eine Ernährungssonde.

Der Nahrungsbehälter kann jeglicher für die enterale Ernährung geeignete Behälter sein. Dem Fachmann sind zahlreiche solcher Nahrungsbehälter bekannt, die typischerweise in Beutelform oder als semirigide Behälter vorliegen und vorzugsweise aus Kunststoff hergestellt werden.

Dem Fachmann sind ebenfalls verschiedene geeignete Ausführungsformen von Verbindungsstutzen bekannt, die alle als von der vorliegenden Erfindung umfasst angesehen werden sollen. Der Verbindungsstutzen wird auch als "Port" bezeichnet und dient in einer bevorzugten Ausführungsform als Entnahme- und/oder Einfüllteil. Der Verbindungsstutzen kann demnach geeignet sein, den Nahrungsbehälter mit Nahrung zu füllen und/oder diese aus dem Nahrungsbehälter zu entnehmen. Eine weitere Funktion des Verbindungsstutzens besteht darin, einen Kanal für den Spike bereitzustellen. Der Verbindungsstutzen stelltein Befestigungsmittel für den Aufsatz bereit.

In einer Ausführungsform besteht der Verbindungsstutzen aus einer Verbindungskomponente, die mit der Beutelfolie veklebt oder verschweißt wird und einem hohlzylinderförmigen, vorzugsweise steifen Teil, wobei der hohlzylinderförmige Teil mit dem Aufsatz in Verbindung ist.

Die Verbindung zwischen Verbindungsstutzen und Nahrungsbehälter kann auf jede dem Fachmann geläufige Art und Weise erreicht werden. In einer bevorzugten Ausführungsform ist der Verbindungsstutzen direkt auf die Wand des Nahrungsbehälters aufgebracht, z.B. durch Aufkleben. Hierdurch besteht zunächst kein direkter Zugang zu der im Behälter enthaltenen Nahrung, d.h. die Nahrungsbehälterwand selbst dient als Versiegelung. In einer weiteren bevorzugten Ausführungsform ist der Verbindungsstutzen in die Beutelnaht des Nahrungsbehälters eingeschweißt. Dies hat den Vorteil, dass eine sicherere Befestigung des Verbindungsstutzens mit dem Nahrungsbehälter erreicht wird und der Beutel durch den Verbindungsstutzen befüllt werden kann.

Der Verbindungsstutzen weist in einer weiteren Ausführungsform zumindest ein Versieglungsmittel auf. Ein solches Versiegelungsmittel hat den Zweck und Vorteil, dass eine Kontamination der im Behälter enthaltenen Nahrung vermieden wird. Obgleich alle dem Fachmann bekannten und geeigneten Versieglungsmittel als von der Erfindung umfasst angesehen werden, ist das Versieglungsmittel in einer bevorzugten Ausführungsform eine Membran, Folie oder Aluminiumfolie welche auf der dem Nahrungsbehälter abgewandten Seite des Verbindungsstutzens aufgebracht wird. Noch bevorzugter ist das Versieglungsmittel eine aufsteckbare oder aufschraubbare Verschlusskappe, welche auf auf der dem Nahrungsbehälter abgewandten Seite des Verbindungsstutzens aufgebracht wird. Diese Verschlußkappe enthält eine Membran die einerseits als Dichtelement zwischen Verschußkappe und Verbindungsstutzen dient, die anderseits aber auch eine Ventilfunktion aufweist. Eine solche Membran erlaubt es, den Zugang zu der im Behälter befindlichen Nahrung reversibel zu öffnen und zu schließen. Dies wird beispielsweise durch eine Membran mit sternförmigen, zentralen Einschneidungen ermöglicht, die nach dem Zurückziehen des Spikes die Öffnung zur Nahrung verschließen. Eine solche Membran bietet den Vorteil, dass ein Nahrungsmittelbeutel von der Überleiteinheit entfernt und erneut angeschlossen werden kann, insbesondere dann, wenn der Nahrungsbehälter an einem Infusionsständer hängt. Dem Fachmann ist eine Vielzahl solcher Membranen mit reversibler Ventilfunktion bekannt, beispielsweise die in den von Fresenius Kabi unter dem Namen EasyBag vertriebenen enteralen Nahrungsbeuteln enthaltene Membran. In einer weiteren bevorzugten Ausführungsform weist der Verbindungsstutzen eine innenliegende Membran auf, bevorzugt mit reversibler Ventilfunktion, und eine über der Membran liegende versiegelnde Folie, bevorzugt eine Aluminiumfolie.

Die Überleiteinheit kann jede geeignete Überleiteinheit zum Zwecke der enteralen Ernährung sein. Beispielhafte Überleiteinheiten sind kommerziell von Fresenius Kabi unter dem Handelsnamen "APPLIX" erhältlich. Die Überleiteinheit stellt das Verbindungsglied zwischen Nahrungsbeutel und Ernährungssonde dar und weist einen Sondenschlauch mit einem distalen und proximalen Verbindungsstück auf. Über das distale Verbindungsstück ist sie mit der Ernährungssonde verbindbar und über das proximale Verbindungsstück ist sie über den Aufsatz mit dem Nahrungsbehälter verbunden. Die Überleiteinheit umfasst bevorzugt eine Pumpe zum Transport der Nahrung aus dem Nahrungsbehälter durch die Ernährungssonde in den Patienten. Die Überleiteinheit ist bevorzugt ein DEHP-freies System. Die Ernährungssonde kann jede dem Fachmann bekannte Ernährungssonde sein, bevorzugt eine Magensonde oder eine PEG-Sonde.

Der Aufsatz dient der Herstellung der Verbindung zwischen Verbindungsstutzen und Überleiteinheit. Diese Verbindung wird über die zumindest zwei Verbindungsstücke des Aufsatzes erreicht. Die Verbindungsstücke sind derart ausgebildet, dass ein Nahrungsfluss zwischen Verbindungsstutzen und Überleiteinheit ermöglicht wird. Zu diesem Zweck weisen die Verbindungsstücke vorzugsweise jeweils zumindest eine Öffnung auf, wobei die Öffnungen der Verbindungsstücke beispielsweise durch einen als Kanal ausgebildeten Hohlraum miteinander in Verbindung stehen und den Fluss der Nahrung von einer Öffnung zur anderen erlauben.

In einer bevorzugten Ausführungsform weist der erfindungsgemäße Aufsatz genau zwei Verbindungsstücke auf. In einer weiteren bevorzugten Ausführungsform weist der Aufsatz 3, 4, 5, oder mehr als 5 Verbindungsstücke auf, wobei zumindest ein Verbindungsstück mit dem Überleitsystem und zumindest ein Verbindungsstück mit dem Verbindungsstutzen verbunden ist. Noch bevorzugter sind bei mehr als 2 Verbindungsstücken alle bis auf eines mit dem Verbindungsstutzen eines Nahrungsbehälters verbindbar. Dies hat unter anderem den Vorteil, dass der Patient gleichzeitig über eine einzige Ernährungssonde mit möglicherweise unterschiedlicher Nahrung aus mehr als einem Nahrungsbehälter versorgt werden kann.

Der Aufsatz kann aus jeglichem geeigneten Material hergestellt sein, wobei Kunststoffe bevorzugt sind.

In einer weiteren bevorzugten Ausführungsform ist zumindest ein Verbindungsstück des Aufsatzes als Dichtung ausgebildet, die bevorzugt eine sichere und formschlüssige Verbindung zwischen Aufsatz und Überleitsystem und/oder Aufsatz und Verbindungsstutzen herstellt. Eine Dichtung verhindert vorteilhaft das Austreten von Nahrung und die Kontamination der Nahrung über die Verbindungsstelle zwischen Aufsatz und Verbindungsstutzen und/oder Aufsatz und Überleiteinheit.

Das mit dem Verbindungsstutzen verbundene indbare erste Verbindungsstück ist so ausgebildet, dass eine irreversible Verbindung zwischen Aufsatz und Verbindungsstutzen erfolgt. Bevorzugt werden die Nahrungsbehälter direkt mit dem verbundenen Aufsatz vom Hersteller ausgeliefert. Dies hat den Vorteil, dass es dem Benutzer eines solchen Nahrungsbehälters mit bereits aufgesetztem Aufsatz nicht mehr möglich ist, eine Überleiteinheit, welche als Verbindungselement einen Spike aufweist, über den Verbindungsstutzen an den Nahrungsbehälter anzuschließen. So wird beispielsweise der Anschluss einer Überleiteinheit verhindert, die für intravenösen Applikation verwendet wird.

Dem Fachmann ist eine Vielzahl von Möglichkeiten bekannt, eine irreversible Verbindung zwischen Aufsatz und Verbindungsstutzen zu gewährleisten. Unter dem Begriff "irreversible Verbindung" wird vorliegend bevorzugt verstanden, dass der Anwender die Verbindung nicht ohne den Einsatz von übermäßiger Kraft und/oder den Einsatz von Werkzeug lösen kann. In bevorzugten Ausführungsformen weist entweder der Aufsatz oder der Verbindungsstutzen eine Einrastkante auf, die mit einem korrespondierenden Vorsprung des zu verbindenden Verbindungsstutzens bzw. Aufsatzes eine irreversible Verbindung eingeht. Dies hat den Vorteil, dass der Aufsatz einfach über den Verbindungsstutzen gestülpt werden kann - oder umgekehrt - und sofort eine irreversible Verbindung hergestellt wird. Weitere Alternativen oder zusätzliche Mittel eine irreversible Verbindung herzustellen sind Verkleben und/oder Verschweißen.

In einer besonders bevorzugten Ausführungsform der Erfindung wird eine irreversible Verbindung zwischen Verbindungsstutzen des Nahrungsbehälters und dem Aufsatz bereits vor der Auslieferung der Nahrungsmittelbehälter an den Verbraucher/Anwender durchgeführt. Dies hat den Vorteil, dass sichergestellt wird, dass vom Verbraucher/Anwender immer ein zum zweiten Verbindungsstück des Aufsatzes komplementärer Konnektor verwendet werden muss, um den Nahrungsbehälter zu öffnen. Somit wird daher sichergestellt, dass nur Verbindungslösungen ohne Spike Verwendung finden.

In einer anderen bevorzugten Ausführungsform wird eine irreversible Verbindung zwischen dem Aufsatz und dem Verbindungsstutzen mittels eines Klebstoffes erreicht, der auch zusätzlich verwendet werden kann.

Das mit der Überleiteinheit verbundene zweite Verbindungsstück ist bevorzugt so ausgebildet, dass eine einfach herstellbare und/oder lösbare Verbindung mit dem Überleitelement herstellbar ist. Noch bevorzugter weist dieses zweite Verbindungsstück ein Arretierungselement auf, das derart ausgebildet ist, dass eine formschlüssige und lösbare Verbindung mit einem Verbindungsstück der Überleiteinheit herstellbar ist, welches ein mit dem Arretierungselement des zweiten Verbindungsstücks komplementäres Arretierungselement aufweist. Das Arretierungselement kann jede dem Fachmann bekannte und geeignete Ausgestaltung aufweisen. Als Arretierungselemente kommen grundsätzlich alle formschlüssigen lösbaren Verbindungen in Frage, beispielsweise Steckverbindungen und Schraubverbindungen, insbesondere Überwurfkappen, Schraubverschlüsse, Bajonettverschlüsse oder dergleichen.

In einer besonders bevorzugten Ausführungsform ist das Arretierungselement des zweiten Verbindungsstücks als eine Schraubkappe mit einem Innengewinde ausgebildet.

In einer weiteren besonders bevorzugten Ausbildungsform ist das zweite Verbindungsstück des Aufsatzes konisch oder mit mehreren Abschnitten unterschiedlichen Durchmessers stufenförmig ausgebildet und weist ein Arretierungselement auf, dass derart ausgebildet ist, dass einerseits eine formschlüssige lösbare Verbindung mit einem Verbindungsstück einer Überleiteinheit herstellbar ist, das ein mit dem Arretierungselement des zweiten Verbindungsstücks komplementäres Arretierungselement aufweist, und durch dass andererseits eine lösbare Verbindung mit trichterförmigen Verbindungsstücken unterschiedlichen Durchmessers von verschiedenen Überleiteinheiten herstellbar ist.

Noch bevorzugter ist das zweite Verbindungsstück des Aufsatzes als männlicher oder weiblicher "ENLock"-Konnektor ausgebildet. Dies ermöglicht eine schnelle und sichere Verbindung mit der Überleiteinheit, falls deren Verbindungsstück als komplementärer weiblicher bzw. männlicher "ENLock"-Konnektor ausgebildet ist. Auch ein schnelles und bequemes Lösen einer solchen Verbindung wird hierdurch ermöglicht. Das "ENLock" Verbindungssystem ist dem Fachmann hinlänglich bekannt und wird unter anderem in der WO 2008/049568 A beschrieben. die als Entwurf der ISO Arbeitgruppe - TC210 / JWG4, PG 3 - Enteral Connectors - vorliegt und einsehbar ist.

Die Ausbildung des zweiten Verbindungsstücks des Aufsatzes als "ENLock"-Konnektor bietet neben der einfach zu handhabenden und sicheren Verbindung unter anderem den Vorteil, dass erhebliche Kosteneinsparungen bei den Überleiteinheiten möglich sind, und dass die Handhabung der Überleiteinheit erleichtert wird, da hier zu dem bereits distal vorhandenen "ENLock"-Konnektor lediglich ein weiterer "ENLock"-Konnektor als proximales Verbindungselement eingesetzt werden zu braucht und somit die häufig auszutauschenden Überleiteinheiten keine Mittel zum Öffnen des Nahrungsbehälters mehr umfassen müssen.

In einer weiteren bevorzugten Ausführungsform umfasst der Aufsatz weiterhin zumindest ein Mittel zum Versiegeln zumindest eines der Verbindungsstücke, bevorzugt aller Verbindungsstücke. Dies hat den Vorteil, dass das Innere des Aufsatzes steril gehalten werden kann. Noch bevorzugter umfasst der Aufsatz ein Mittel zur Versieglung des zweiten Verbindungsstücks. Dies hat den Vorteil, dass der Nahrungsbehälter nach Aufsetzen des Aufsatzes und vor der Verbindung mit der Überleiteinheit verschlossen ist und eine Kontamination der Nahrung verhindert wird. Dem Fachmann ist eine Vielzahl von möglichen Versieglungsmitteln bekannt, die zur Versieglung des zweiten Verbindungsstücks verwendet werden kann. Bevorzugte Mittel schließen Steckverbindungen und Schraubverbindungen, Überwurfkappen, Schraubverschlüsse, Bajonettverschlüsse, Membranen, Folien, insbesondere Aluminiumfolie, Aufsteck-, Aufschraub- und/oder Abreißkappen ein.

Der Aufsatz enthält weiterhin ein Mittel zum Öffnen des Nahrungsbehälters. Vorzugsweise ist das Mittel zum Öffnen in den Aufsatz integriert, noch bevorzugter ist es nach Verbindung von Aufsatz mit Verbindungsstutzen von außen nicht mehr sichtbar und/oder zugänglich.

Das Mittel zum Öffnen des Nahrungsbehälters kann jedes geeignete Mittel sein. In einer bevorzugten Ausführungsform ist es ein Mittel zum Durchstechen einer Versiegelung des Nahrungsbehälters, noch bevorzugter ist es ein Spike, bzw. ein oder mehrere Dorne, oder eine Kanüle. Wie eingangs erwähnt, sind Nahrungsbehälter verschiedener Hersteller verfügbar, die sich hinsichtlich der Versieglung und damit des Verfahrens zu ihrer Öffnung unterscheiden. Dementsprechend wird auch das Mittel zum Öffnen des Nahrungsbehälters verschieden ausgeführt sein. Der Fachmann wird, ausgehend von der jeweiligen Versieglung des Nahrungsbehälters, mühelos das Mittel zur Öffnung desselben anpassen können.

In einer Ausführungsform ist der Verbindungsstutzen direkt auf die Nahrungsbehälterwand aufgebracht, wie dies beispielsweise bei den Nahrungsbehältern des Herstellers Nestlé der Fall ist. Hier dient demnach die Nahrungsbehälterwand selbst als Versieglung des Nahrungsbehälters. Dementsprechend muss das Mittel zum Öffnen des Nahrungsbehälters geeignet sein, die die Nahrungsbehälterwand zu durchstechen. Des Weiteren muss das Mittel zum Öffnen eine ausreichende Länge aufweisen, um von der vom Nahrungsbehälter wegweisenden Öffnung des Verbindungsstutzens bis zumindest hinter die Nahrungsbehälterwand zu reichen. In einer bevorzugten Ausführungsform ist das Mittel zum Öffnen des Nahrungsbehälters einstückig mit dem Aufsatz hergestellt und hat die Form einer Kanüle, die, noch bevorzugter, zentral im Inneren des Aufsatzes angeordnet ist.

In einer anderen Ausführungsform ist der Verbindungsstutzen in die Naht der Nahrungsbehälterfolie eingeschweißt, wie dies beispielsweise bei den Nahrungsbehältern der Hersteller Danone oder Fresenius Kabi der Fall ist. In diesen Fällen ist der Verbindungsstutzen am von dem Nahrungsbehälter wegweisenden Ende durch ein Versieglungsmittel versiegelt, vorzugsweise durch eine Membran und/oder einer Folie, wie Aluminiumfolie. Dementsprechend muss das Mittel zum Öffnen des Nahrungsbehälters geeignet sein, diese Versieglung(en) zu durchstechen. Des Weiteren muss das Mittel zum Öffnen im Vergleich zu der vorstehenden Ausführungsform lediglich eine geringere Länge aufweisen, um von der vom Nahrungsbehälter wegweisenden Öffnung des Verbindungsstutzens bis zumindest hinter die Versiegelung zu reichen. In einer bevorzugten Ausführungsform ist das Mittel zum Öffnen des Nahrungsbehälters einstückig mit dem Aufsatz hergestellt und hat die Form eines Spikes, bzw. eines oder mehrerer Dorne oder einer Kanüle, die, noch bevorzugter, zentral im Inneren des Aufsatzes angeordnet ist.

In einer weiteren Ausführungsform ist das Mittel zum Öffnen des Nahrungsbehälters in Richtung des ersten Verbindungsstücks bewegbar, d.h. in Richtung der Versieglung des Nahrungsbehälters. Bevorzugt ist diese Bewegung eine axiale Bewegung. Das Mittel zum Öffnen des Nahrungsbehälters kann zumindest zwei definierte Positionen einnehmen: eine erste, in welcher es die Versieglung des Nahrungsbehälters nicht durchdringt, d.h. der Nahrungsbehälter verschlossen ist, und eine zweite, in welcher es die Versieglung des Nahrungsbehälters durchdringt, d.h. der Nahrungsbehälter geöffnet ist. Es sind neben diesen beiden auch weitere Positionen denkbar, z.B. zwischen diesen beiden genannten Punkten liegende, oder z.B. durch eine Schraubverbindung zwischen Aufsatz und Verbindungsstutzen erzeugte stufenlose Positionen.

Der Aufsatz kann weiterhin ein Mittel zur Arretierung des Mittels zur Öffnung des Nahrungsbehälters umfassen, welches das Mittel zum Öffnen in zumindest einer der zumindest zwei definierten Positionen hält. Bevorzugte solcher Mittel zur Arretierung sind bereits weiter oben beschrieben worden.

In einer besonders bevorzugten Ausführungsform weist der Aufsatz ein Gewinde auf, das die Bewegung des Mittels zum Öffnen durch eine Drehbewegung des Aufsatzes ermöglicht. Das Gewinde des Aufsatzes greift hierbei vorzugsweise in ein komplementäres Gewinde ein, welches vom Verbindungsstutzen umfasst wird. Dies hat den Vorteil, dass der Nahrungsbehälter einfach und sicher zu öffnen ist. Insbesondere stellt ein Gewinde gleichzeitig ein Mittel zur Arretierung des Mittels zum Öffnen des Nahrungsbehälters bereit. Das vom Aufsatz umfasste Gewinde kann ein Innen- oder Außengewinde sein, bevorzugt ist es ein Innengewinde. Das Gewinde des Verbindungsstutzens kann dementsprechend ein Außen- oder Innengewinde sein und ist bevorzugt ein Außengewinde.

Beschrieben wird auch ein Aufsatz für eine Vorrichtung zur enteralen Ernährung, der zumindest zwei Verbindungsstücke umfasst, wobei das erste Verbindungsstück mit dem Verbindungsstutzen eines Nahrungsbehälters verbindbar ist und das zweite Verbindungsstück mit einer Überleiteinheit verbindbar ist, sodass ein Nahrungsfluss von Nahrungsbehälter durch Verbindungsstutzen und Aufsatz zur Überleiteinheit ermöglicht wird, wobei das zweite Verbindungsstück als "ENLock"-Konnektor ausgebildet ist. Bereits im Produktionsprozess kann ein Nahrungsbeutel mit an dessen Verbindungsstutzen irreversibel verbundenem Aufsatz hergestellt werden. Somit ist sichergestellt, dass der Verbraucher/Anwender immer einen zum zweiten Verbindungsstück des Aufsatzes komplementären Konnektor verwenden muss, um den Nahrungsbehälter zu öffnen. Somit wird daher sichergestellt, dass nur Verbindungslösungen ohne Spike Verwendung finden.

Dieser beschriebene Aufsatz, der nicht zur Erfindung gehört, weist demnach kein Mittel zur Öffnung des Nahrungsbehälters auf, kann aber alle ansonsten oben beschriebenen Merkmale verwirklichen. Ein solcher Aufsatz ist beispielsweise für Nahrungsmittelbehälter geeignet, die keine zu durchstechende Versieglung aufweisen. So kann der Verbindungsstutzen des Nahrungsbehälters lediglich durch einen entfernbaren Verschluss auf dem Verbindungsstutzen geschlossen sein, wie z.B. ein Schraubverschluss, eine Abreißkappe und/oder eine aufgebrachte Folie. Nach Entfernung dieser Versieglung durch den Benutzer ist damit bei einem in die in die Beutelnaht eingeschweißten Verbindungsstutzen bereits der direkte Zugang zur Nahrung gegeben. In einem solchen Fall kann zwar auch der Aufsatz mit dem Mittel zum Öffnen des Nahrungsbehälters verwendet werden, dies ist jedoch nicht unbedingt nötig. Der Aufsatz ohne Mittel zum Öffnen des Nahrungsmittelbehälters aber mit als "ENLock"-Konnektor ausgebildetem zweiten Verbindungsstück hat daher die Vorteile auf der einen Seite eine Kostenreduktion des Aufsatzes zu erzielen, auf der anderen Seite aber immer noch zu gewährleisten, dass es aufgrund des "ENLock"-Konnektors einem Benutzer nicht möglich ist, andere im klinischen Umfeld verwendete Verbindungstypen - beispielsweise bei intravenösen Applikation -, die einen Spike umfassen, mit dem Nahrungsbehälter zu verbinden.

In einer Ausführungsform weist der Verbindungsstutzen der Vorrichtung zumindest ein Versieglungsmittel auf, welches auf der dem Nahrungsbehälter abgewandte Seite des Verbindungsstutzens angeordnet ist und schützt damit den Inhalt des Nahrungsbehälters vor Kontamination. In einer bevorzugten Ausführungsform ist das zumindest eine Versieglungsmittel eine Membran, Folie, Aluminiumfolie, Aufsteck-, Aufschraub- und/oder eine Abreißkappe. In einer weiteren bevorzugten Ausführungsform weist der Verbindungsstutzen eine innenliegende Membran, bevorzugt mit reversibler Ventilfunktion, und eine über der Membran liegende Folie, bevorzugt eine Aluminiumfolie auf.

In einer weiteren bevorzugten Ausführungsform weisen der Aufsatz und der Verbindungsstutzen der Vorrichtung jeweils zumindest ein Gewinde auf, die ineinandergreifen und somit die Bewegung des Mittels zum Öffnen in Richtung des ersten Verbindungsstücks ermöglichen. Bevorzugt wird das Mittel zum Öffnen hierbei axial bewegt und/oder die Bewegung durch eine Drehbewegung des Aufsatzes ermöglicht. In einer bevorzugten Ausführungsform kann die Axialbewegung auch mit einer Bajonettverbindung erzeugt und/oder arretiert werden.

In einer ebenfalls bevorzugten Ausführungsform umfasst die Vorrichtung zur enteralen Ernährung weiterhin eine Überleiteinheit, wobei die Überleiteinheit und der Aufsatz formschlüssig über das zweite Verbindungsstück des Aufsatzes miteinander verbunden sind. Noch bevorzugter sind sowohl das zweite Verbindungsstück des Aufsatzes als auch das damit verbundene Verbindungsstück der Überleiteinheit als komplementärer "ENLock"-Konnektor ausgebildet. Beispielsweise ist das zweite Verbindungsstück des Aufsatzes ein männlicher "ENLock"-Konnektor, während das Verbindungsstück der Überleiteinheit ein weiblicher "ENLock"-Konnektor ist, oder umgekehrt.

Die Verwendung der zuvor beschriebenen erfindungsgemäßen Vorrichtung umfassend den Aufsatz für die enterale Ernährung eines Individuums, z.B. eines Menschen oder eines Tieres, wird auch beschrieben.

Ein Verfahren zur Bereitstellung einer Vorrichtung zur enteralen Ernährung, umfasst den Schritt des Verbindens eines Nahrungsbehälters und einer Überleiteinheit mittels des Aufsatzes.

Die Überleiteinheit kann mit dem Aufsatz verbunden werden, nachdem der Nahrungsbehälter mittels des in dem Aufsatz enthaltenen Mittels zur Öffnung geöffnet wurde. Hierbei kann vor dem Verbinden mit der Überleiteinheit und trotz des Durchstoßens des Versieglungsmittel des Nahrungsbehälters, d.h. trotz der Öffnung des Nahrungsbehälters ein Schutz der im Behälter befindlichen Nahrung vor Kontamination gewährleistet werden, falls das zweite Verbindungsstück des Aufsatzes versiegelt ist, beispielsweise durch eine Abreißkappe, einen Schraubverschluss, oder eine Aluminiumfolie.

Die Überleiteinheit kann mit dem Aufsatz verbunden werden, bevor der Nahrungsbehälter mittels des in dem Aufsatz enthaltenen Mittels zur Öffnung geöffnet wurde, bevorzugt aber nachdem der Aufsatz mit dem Nahrungsbehälter verbunden wurde. Dies hat den Vorteil, dass bereits ein gegenüber der Außenwelt geschlossenes Gesamtsystem hergestellt ist, bevor der Nahrungsfluss einsetzt.

Im Folgenden werden verschiedene Ausführungsbeispiele der Erfindung unter Bezugnahme auf die Zeichnungen näher erläutert.

### Kurze Beschreibung der Zeichnungsfiguren

Es zeigen:

Fig. 1 den schematischen Aufbau zweier gebräuchlicher Varianten von Nahrungsbehältern.

Fig. 2 eine erste Ausführungsform der erfindungsgemäßen Vorrichtung.

Fig. 3 eine zweite Ausführungsform der erfindungsgemäßen Vorrichtung.

Fig. 4 eine dritte Ausführungsform der erfindungsgemäßen Vorrichtung.

Fig. 5 eine vierte Ausführungsform der erfindungsgemäßen Vorrichtung.

### Beschreibung der Ausführungsarten

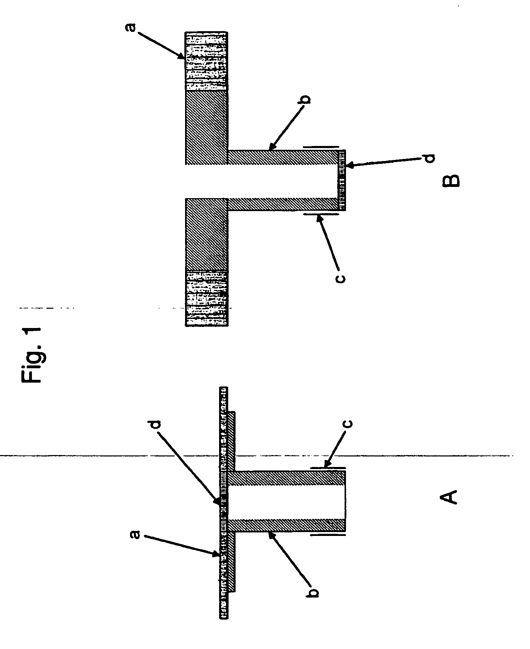

In Fig. 1 ist der der schematische Aufbau zweier gebräuchlicher Varianten von Nahrungsbehältern zu erkennen. Gezeigt ist ein Ausschnitt aus dem Bereich eines Nahrungsbeutels, in welchem der Verbindungsstutzen mit dem Nahrungsbeutel in Verbindung gebracht wird.

In Fig. 1A wird ein Schema der Nahrungsbeutel des Herstellers Nestlé gezeigt. Zu erkennen ist die Nahrungsbeutelwand a, auf die der Verbindungsstutzen b aufgeklebt ist. Der Verbindungsstutzen dient als Kanal für das Mittel zum Öffnen und als Entnahmeteil und weist ein Gewinde c auf, über welches das in den Aufsatz integrierte Mittel zum Öffnen der Versiegelung axial verschiebbar ist. Die irreversible Verbindung des Aufsatzes mit dem Verbindungsstutzen wird bevorzugt über eine Schnappverbindung erreicht, die in Fig 2f gezeigt ist.

Zu erkennen ist weiterhin der Abschnitt d des Nahrungsbeutels, der vom Mittel zum Öffnen des Aufsatzes durchdrungen werden muss, um den Nahrungsbeutel zu Öffnen und die Entnahme der Nahrung zu gewährleisten. Im hier gezeigten Beispiel ist demnach kein weiteres Versieglungsmittel vorhanden, da die Nahrungsbeutelwand selbst das Versieglungsmittel darstellt.

In Fig. 1B wird ein Schema der Nahrungsbeutel des Herstellers Danone gezeigt. Zu erkennen ist wiederum die Nahrungsbeutelwand a, die eine Schweißnaht aufweist, in welche der Verbindungsstutzen b eingeschweißt wurde. Der Verbindungsstutzen dient als Einfüll- und Entnahmeteil und weist ein Gewinde c auf, über welches das in den erfindungsgemäßen Aufsatz integrierte Mittel zum Öffnen der Versiegelung axial verschiebbar ist. Die irreversible Verbindung des Aufsatzes mit dem Verbindungsstutzen wird bevorzugt über eine Schnappverbindung erreicht, die in Fig 2f gezeigt ist.

Zu erkennen ist weiterhin die Versieglung d des Nahrungsbeutels, der vom Mittel zum Öffnen des Aufsatzes durchdrungen werden muss, um den Nahrungsbeutel zu öffnen und die Entnahme der Nahrung zu gewährleisten. Im hier gezeigten Beispiel wurde eine versiegelnde Membran auf das vom Nahrungsbeutel wegweisende Ende des Verbindungsstutzens aufgebracht, um eine Kontamination des Beutelinhalts zu vermeiden.

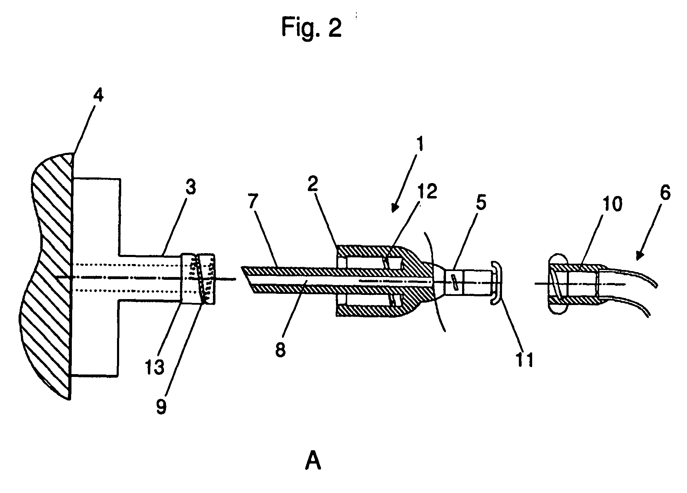

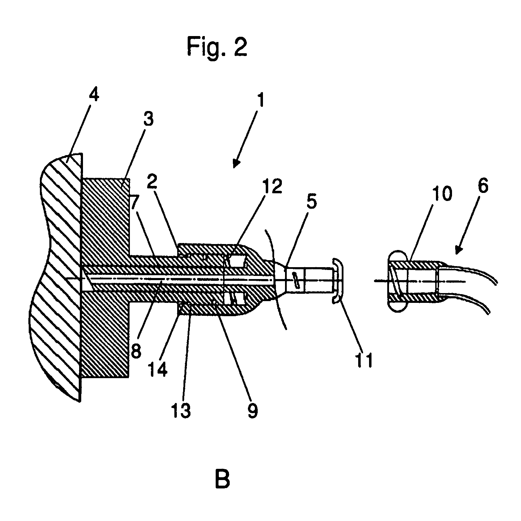

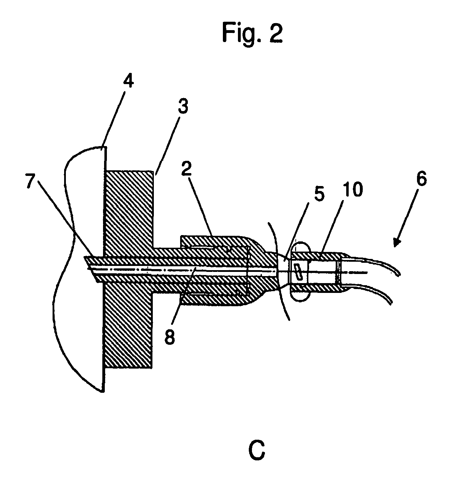

Fig. 2 zeigt eine erste Ausführungsform der erfindungsgemäßen Vorrichtung.

Wie in Fig. 2A zu erkennen ist, umfasst der Aufsatz 1 für eine Vorrichtung zur enteralen Ernährung die zwei Verbindungsstücke 2 und 5. Das erste Verbindungsstück 2 ist mit dem Verbindungsstutzen 3 eines Nahrungsbehälters 4 verbunden. Das zweite Verbindungsstück 5 ist mit der Überleiteinheit 6 verbunden, wobei dieses Verbindungsstück 5 des Aufsatzes als männlicher "ENLock"-Konnektor ausgebildet ist und das Verbindungsstück 10 der Überleiteinheit, welches am proximalen Ende des Sondenschlauchs der Überleiteinheit angebracht ist, als weiblicher "ENLock"-Konnektor ausgebildet ist. Das zweite Verbindungsstück 5 des Aufsatzes weist weiterhin eine Versieglung in Form einer Abreißkappe 11 auf.

Wie zu erkennen ist, umfasst der Nahrungsbehälter kein weiteres Versieglungsmittel, sondern der Verbindungsstutzen ist auf die Nahrungsbehälterwand aufgeklebt.

Der Aufsatz weist weiterhin ein Mittel zum Öffnen des Nahrungsbehälters 7 auf, welches als längerer Spike ausgebildet ist und einen die beiden Verbindungsstücke 2 und 5 verbindenden Hohlraum bzw. Kanal 8 aufweist, sodass ein Nahrungsfluss vom Nahrungsbehälter durch Verbindungsstutzen und Aufsatz zur Überleiteinheit ermöglicht wird. Aufsatz 1 und das Mittel zum Öffnen sind einstückig aus Kunststoff hergestellt.

Der Verbindungsstutzen 3 weist weiterhin ein Außengewinde 9 auf, in welches das Innengewinde 12 des Aufsatzes eingreift. Somit kann durch eine Rotation des Aufsatzes das Mittel zum Öffnen des Nahrungsbehälters 7 axial in Richtung des ersten Verbindungsstücks bewegt werden und nach Durchstoßen der Nahrungsbehälterwand arretiert werden.

Der Verbindungsstutzen 3 weist weiterhin einen Vorsprung 13 auf, der mit einer Einrastkante des Aufsatzes korrespondiert und eine irreversible Schnappverbindung zwischen Verbindungsstutzen und Aufsatz ermöglicht, die im Produktionsprozess hergestellt wird, sodass der Kanal des Verbindungstutzes nicht für eine Konnektion mit dem Spike einer intravenös einzusetzenden Überleiteinheit verbunden werden kann. Weiterhin wird somit die Verbindung zwischen Verbindungsstutzen und Aufsatz abgedichtet und der Inhalt des Nahrungsbehälters wirksam vor Kontamination geschützt.

Fig. 2B zeigf dieselbe Ausführungsform wie Fig. 2A, nachdem der Aufsatz 1 mit dem Verbindungsstutzen 3 verbunden wurde. Die Verbindung wurde durch einfaches Aufstecken des Aufsatzes auf den Verbindungsstutzen im Produktionsprozess erreicht, wobei die Einrastkante 14 des Aufsatzes hinter den Vorsprung 13 des Verbindungsstutzen gedrückt wurde, um eine irreversible Bindung zu gewährleisten.

Weiterhin zu erkennen ist, dass das Innengewinde 12 des Aufsatzes noch nicht vollständig in das Außengewinde 9 des Verbindungsstutzens eingreift und das Mittel zum Öffnen des Nahrungsbehälters 7 nur in einer ersten definierten Position an der Wand des Nahrungsbehälters 4 anliegt, ohne diese zu durchstechen. Außerdem ist die Abreißkappe 11 noch am zweiten Verbindungsstück 5 befestigt.

Fig. 2C zeigt dieselbe Ausführungsform wie Fig. 2A und Fig. 2B, nachdem die Abreißkappe 11 vom zweiten Verbindungsstück 5 entfernt wurde und der männliche "ENLock"-Konnektor 5 des Aufsatzes mit dem weiblicher "ENLock"-Konnektor 10 der Überleiteinheit 6 verbunden wurde. Diese Verbindungsherstellung zeichnet sich durch eine erhöhte Verbindungssicherheit bei gleichzeitiger einfacher Handhabung aus. Insbesondere kann die Verbindung der Überleiteinheit mit dem Aufsatz erfolgen, bevor die Beutelfolie geöffnet wird.

Weiterhin wurde durch eine Drehbewegung des Aufsatzes und durch das Ineinandergreifen der beiden Gewinde 9 und 12 das Mittel zum Öffnen des Nahrungsbehälters 7 axial in Richtung des Nahrungsbehälters 4 in eine zweite definierte Position bewegt und penetriert nun die Wand des Nahrungsbehälters. Hierdurch wird ein hermetisch versiegelter Nahrungsfluss aus dem Nahrungsbehälter 4 durch das Mittel zum Öffnen 7 des Aufsatzes 1, weiter über die "ENLock"-Verbindung 5,10 in das Überleitsystem gewährleistet und das Kontaminationsrisiko ist minimiert.

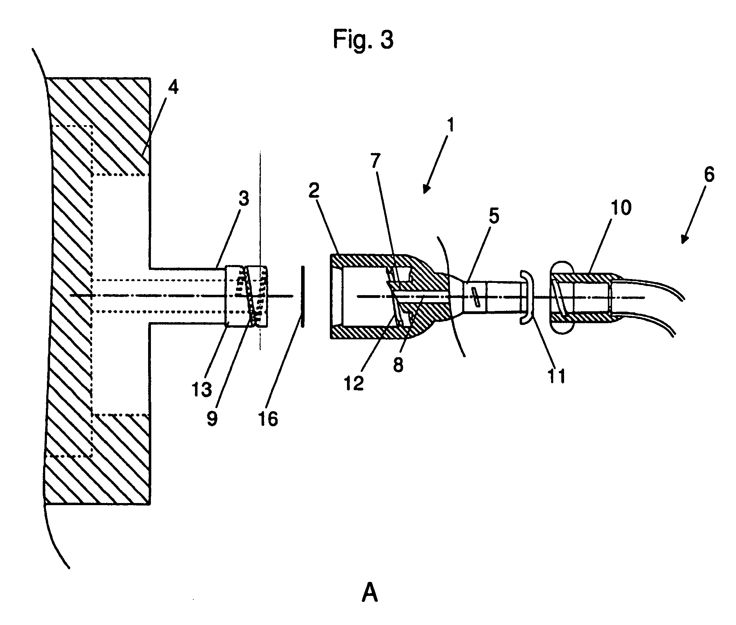

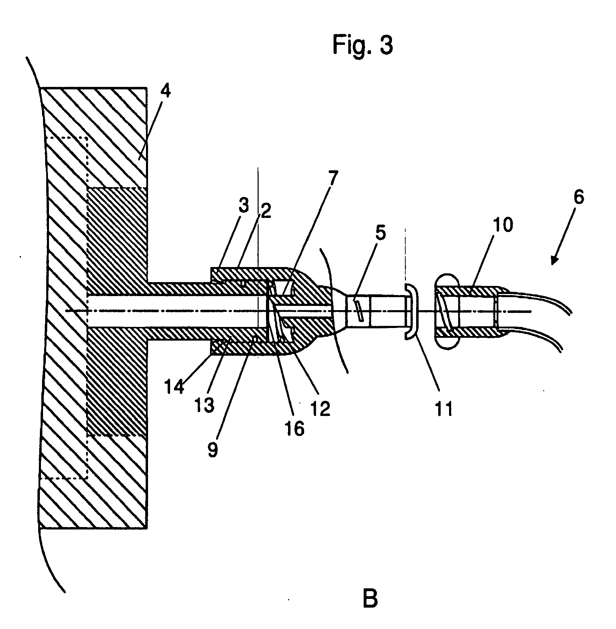

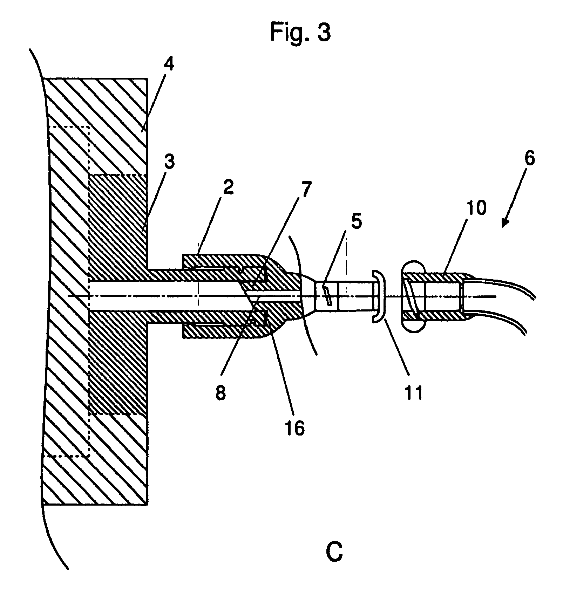

Fig. 3 zeigt eine weitere Ausführungsform der erfindungsgemäßen Vorrichtung.

Wie aus Fig. 3A zu entnehmen ist, umfasst der Aufsatz 1 für eine Vorrichtung zur enteralen Ernährung die zwei Verbindungsstücke 2 und 5. Das erste Verbindungsstück 2 ist mit dem Verbindungsstutzen 3 eines Nahrungsbehälters 4 verbindbar. Diese Verbindung wird irreversibel im Produktiosprozess realisiert. Das zweite Verbindungsstück 5 ist mit der Überleiteinheit 6 verbunden, wobei dieses Verbindungsstück 5 des Aufsatzes als männlicher "ENLock"-Konnektor ausgebildet ist und das Verbindungsstück 10 der Überleiteinheit als weiblicher "ENLock"-Konnektor ausgebildet ist. Das zweite Verbindungsstück 5 des Aufsatzes weist weiterhin eine Versieglung in Form einer Abreißkappe 11 auf.

Wie zu erkennen ist, ist der Verbindungsstutzen 3 mit der Folienschweißnaht des Nahrungsbehälters 4 verschweißt. Daher umfasst der Verbindungsstutzen an der dem Nahrungsbehälter abgewandten Seite als Versieglungsmittel 16 eine versiegelnde Membran.

Der Aufsatz weist weiterhin ein Mittel zum Öffnen des Nahrungsbehälters 7 auf, welches als kurzer Spike ausgebildet ist und einen die beiden Verbindungsstücke 2 und 5 verbindenden Hohlraum bzw. Kanal 8 aufweist, sodass ein Nahrungsfluss von Nahrungsbehälter durch Verbindungsstutzen und Aufsatz zur Überleiteinheit ermöglicht wird. Aufsatz 1 und das Mittel zum Öffnen sind einstückig aus Kunststoff hergestellt.

Der Verbindungsstutzen 3 weist weiterhin ein Außengewinde 9 auf, in welches das Innengewinde 12 des Aufsatzes eingreift. Somit kann auch in dieser Ausführungsform durch eine Rotation des Aufsatzes 1 das Mittel zum Öffnen des Nahrungsbehälters 7 axial in Richtung des ersten Verbindungsstücks 2 bewegt werden und nach Durchstoßen der Nahrungsbehälterwand arretiert werden. Alternativ kann hier auch eine Bajonettmechanik verwendet werden, um die Axialbewegung und deren Arretierung zu erreichen.

Der Verbindungsstutzen 3 weist ebenfalls einen Vorsprung 13 auf, der mit einer Einrastkante des Aufsatzes 1 korrespondiert und eine irreversible Verbindung zwischen Verbindungsstutzen und Aufsatz während des Produktionsprozesses ermöglicht. Weiterhin wird somit die Verbindung zwischen Verbindungsstutzen und Aufsatz abgedichtet und der Inhalt des Nahrungsbehälters wirksam vor Kontamination geschützt.

Fig. 3B zeigt dieselbe Ausführungsform wie Fig. 3A, nachdem der Aufsatz 1 mit dem Verbindungsstutzen 3 verbunden wurde. Die Verbindung wurde durch einfaches Aufstecken des Aufsatzes auf den Verbindungsstutzen im Produktionsprozess erreicht, wobei die Einrastkante 14 des Aufsatzes hinter den Vorsprung 13 des Verbindungsstutzen gedrückt wurde, um eine irreversible Bindung zu gewährleisten.

Weiterhin zu erkennen ist, dass das Innengewinde 12 des Aufsatzes noch nicht vollständig in das Außengewinde 9 des Verbindungsstutzens eingreift und das Mittel zum Öffnen des Nahrungsbehälters 7 in einer ersten definierten Position am Versieglungsmittel 16 anliegt, ohne dieses zu durchstechen. Außerdem ist die Abreißkappe 11 noch am zweiten Verbindungsstück 5 befestigt.

In einem nächsten Schritt kann nun die Abreißkappe entfernt werden und der männliche "ENLock"-Konnektor 5 des Aufsatzes sicher und bequem mit dem weiblichen "ENLock"-Konnektor 10 der Überleiteinheit 6 verbunden werden, um so einen hermetisch versiegelten Nahrungsfluss aus dem Nahrungsbehälter 4 durch das Mittel zum Öffnen 7 des Aufsatzes 1, weiter über die "ENLock"-Verbindung 5,10 in das Überleitsystem bereitzustellen.

Fig. 3C zeigt dieselbe Ausführungsform wie Fig. 3A und Fig. 3B, nachdem durch eine Drehbewegung des Aufsatzes und durch das Ineinandergreifen der beiden Gewinde 9 und 12 das Mittel zum Öffnen des Nahrungsbehälters 7 axial in Richtung des Nahrungsbehälters 4 in eine zweite definierte Position bewegt und das Versieglungsmittel 16 durchstoßen wurde.

Trotz des Durchstoßens des Versieglungsmittels 16, d.h. der Öffnung des Nahrungsbehälters wird ein Schutz der im Behälter befindlichen Nahrung vor Kontamination gewährleistet, da vor dem Durchstoßen des Versiegelungsmittels die Abreißkappe 11 vom zweiten Verbindungsstück 5 entfernt wurde und die Überleiteinheit mittels des "ENLock"-Konnektors angeschlossen wurde.

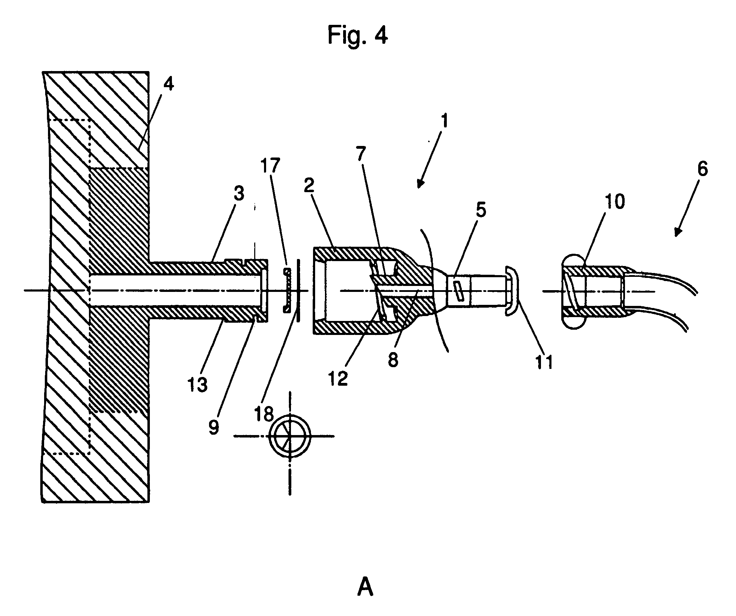

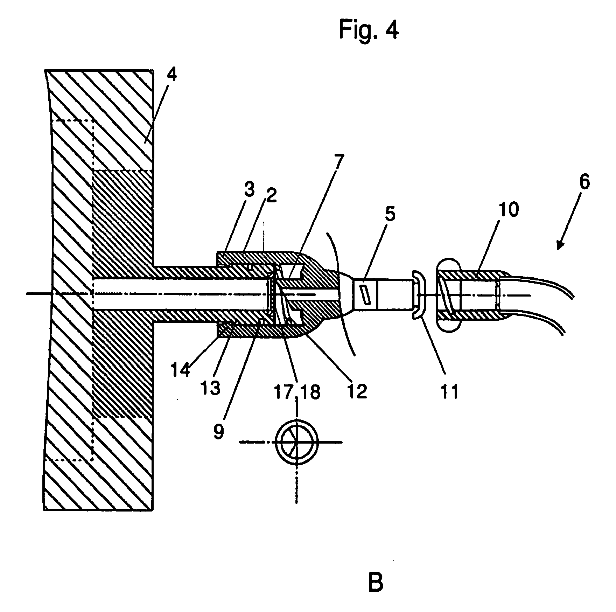

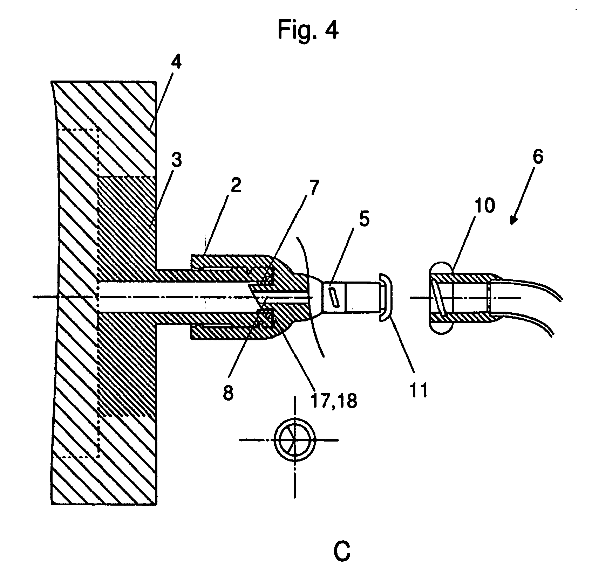

Fig. 4 zeigt eine weitere Ausführungsform der erfindungsgemäßen Vorrichtung. Die in Fig. 4 gezeigte Ausführungsform unterscheidet sich von der in Fig. 3 gezeigten Ausführungsform dadurch, dass eine Membran mit reversibler Ventilfunktion 17 und ein Versiegelungsmittel 18 am Verbindungsstutzen 3 angebracht sind. Die restlichen Bezugszeichen stimmen überein.

Das Membranventil 17 ist eine in das offene Ende des Verbindungsstutzens eingesetzt und besitzt eine reversible Ventilfunktion. Versieglungsmittel 18 ist eine zusätzlich über der Membran angebrachte Aluminiumfolie.

Währende Fig. 4A den Zustand der Vorrichtung vor der Verbindung von Aufsatz 1 mit Verbindungsstutzen 3 wiedergibt, zeigt Fig. 4B dieselbe Ausführungsform, nachdem der Aufsatz 1 mit dem Verbindungsstutzen 3 im Produktionsprozess irrversibel verbunden wurde. Die Verbindung wurde durch einfaches Aufstecken des Aufsatzes auf den Verbindungsstutzen erreicht, wobei die Einrastkante 14 des Aufsatzes hinter den Vorsprung 13 des Verbindungsstutzen gedrückt wurde, um eine irreversible Bindung zu gewährleisten.

Weiterhin zu erkennen ist, dass das Innengewinde 12 des Aufsatzes noch nicht vollständig in das Außengewinde 9 des Verbindungsstutzens eingreift und das Mittel zum Öffnen des Nahrungsbehälters 7 in einer ersten definierten Position am äußeren des Versieglungsmittels 18 anliegt, ohne diese zu durchstechen. Außerdem ist die Abreißkappe 11 noch am zweiten Verbindungsstück 5 befestigt.

Fig. 4C zeigt dieselbe Ausführungsform wie Fig. 3A und Fig. 3B, nachdem durch eine Drehbewegung des Aufsatzes 1 und durch das Ineinandergreifen der beiden Gewinde 9 und 12 das Mittel zum Öffnen des Nahrungsbehälters 7 axial in Richtung des Nahrungsbehälters 4 in eine zweite definierte Position bewegt wurde und Versieglungsmittel 18 sowie Ventilmembran 17 durchstoßen wurden.

Trotz des Durchstoßens des Versieglungsmittel 18, und der Ventilmembran 17, d.h. trotz der Öffnung des Nahrungsbehälters, wird ein Schutz der im Behälter befindlichen Nahrung vor Kontamination gewährleistet, da die Abreißkappe 11 noch nicht vom zweiten Verbindungsstück 5 entfernt wurde. Alternativ, ist die Verbindung der Überleiteinheit mit dem ENLock Konnektor vor Durchstoßen des Versiegelungsmittels 17 denkbar. Das hat den Vorteil, dass bereits ein gegenüber der Aussenwelt geschlossenes Gesamtsytem hergestelllt ist, bevor der Nahrungsfluß einsetzt.

In einem nächsten Schritt kann nun die Abreißkappe entfernt werden und der männliche "ENLock"-Konnektor 5 des Aufsatzes sicher und bequem mit dem weiblichen "ENLock"-Konnektor 10 der Überleiteinheit 6 verbunden werden, um so einen hermetisch versiegelten Nahrungsfluss aus dem Nahrungsbehälter 4 durch das Mittel zum Öffnen 7 des Aufsatzes 1, weiter über die "ENLock"-Verbindung 5,10 in das Überleitsystem bereitzustellen.

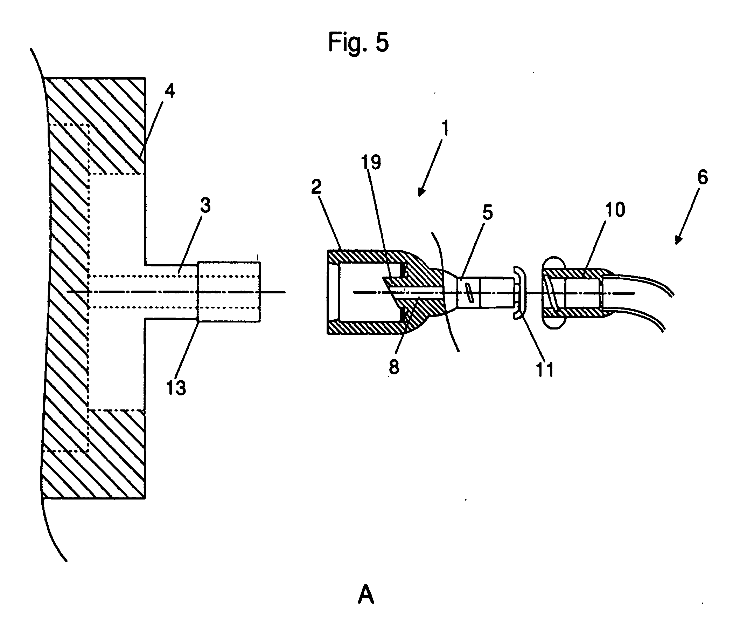

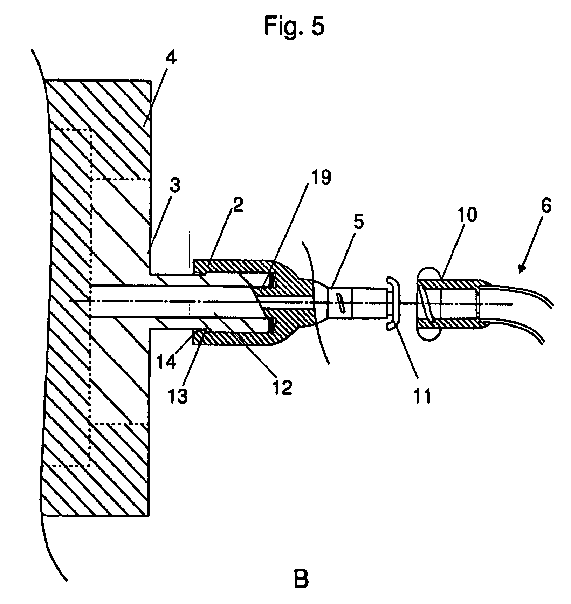

Fig. 5 zeigt noch eine weitere Ausführungsform der erfindungsgemäßen Vorrichtung.

Wie in Fig. 5A dargestellt ist, umfasst der Aufsatz 1 für eine Vorrichtung zur enteralen Ernährung die zwei Verbindungsstücke 2 und 5. Das erste Verbindungsstück 2 ist mit dem Verbindungsstutzen 3 eines Nahrungsbehälters 4 verbunden. Das zweite Verbindungsstück 5 ist mit der Überleiteinheit 6 verbunden, wobei dieses Verbindungsstück 5 des Aufsatzes als männlicher "ENLock"-Konnektor ausgebildet ist und das Verbindungsstück 10 der Überleiteinheit als weiblicher "ENLock"-Konnektor ausgebildet ist. Das zweite Verbindungsstück 5 des Aufsatzes weist weiterhin eine Versieglung in Form einer Abreißkappe 11 auf.

Wie zu erkennen ist, ist der Verbindungsstutzen 3 mit der Wand des Nahrungsbehälters 4 verschweißt und weist einen direkten Zugang zum Inhalt des Nahrungsbehälters auf. Dies kann z.B. deshalb der Fall sein, weil der Verbindungsstutzen zuvor durch eine Versieglung wie beispielsweise einen Abreißkappe gesichert gewesen ist, die bereits entfernt wurde.

Dementsprechend braucht der Aufsatz kein Mittel zum Öffnen des Nahrungsbehälters 7 aufzuweisen, wie dies in den zuvor beschriebenen Ausführungsformen der Fall war. Dennoch ist integriert in den Aufsatz 1 ein kurzer Spike 19 ausgebildet, der einen die beiden Verbindungsstücke 2 und 5 verbindenden Hohlraum bzw. Kanal 8 aufweist, sodass ein Nahrungsfluss von Nahrungsbehälter durch Verbindungsstutzen und Aufsatz zur Überleiteinheit ermöglicht wird. Aufsatz 1 und Spike sind einstückig aus Kunststoff hergestellt.

Der Verbindungsstutzen braucht weiterhin nicht unbedingt ein Außengewinde aufzuweisen, da im Aufsatz kein bewegbares Mittel zum Öffnen des Nahrungsbehälters vorhanden ist. Dennoch kann ein solches Außengewinde gegeben sein, z.B. um einen Schraubverschluss als Versieglungsmittel für den Verbindungsstutzen zu erlauben.

Der Verbindungsstutzen 3 weist einen Vorsprung 13 auf, der mit einer Einrastkante des Aufsatzes 1 korrespondiert und eine irreversible Verbindung zwischen Verbindungsstutzen und Aufsatz ermöglicht. Weiterhin wird somit die Verbindung zwischen Verbindungsstutzen und Aufsatz abgedichtet und der Inhalt des Nahrungsbehälters wirksam vor Kontamination geschützt.

Fig. 5B zeigt dieselbe Ausführungsform wie Fig. 5A, nachdem der Aufsatz 1 mit dem Verbindungsstutzen 3 verbunden wurde. Die Verbindung wurde erneut durch einfaches Aufstecken des Aufsatzes auf den Verbindungsstutzen erreicht, wobei die Einrastkante 14 des Aufsatzes hinter den Vorsprung 13 des Verbindungsstutzen gedrückt wurde, um eine irreversible Bindung zu gewährleisten.

Weiterhin ist die Abreißkappe 11 noch am zweiten Verbindungsstück 5 befestigt, sodass trotz des geöffneten Nahrungsbehälters ein Schutz der im Behälter befindlichen Nahrung vor Kontamination gewährleistet wird.

In einem nächsten Schritt kann nun die Abreißkappe entfernt werden und der männliche "ENLock"-Konnektor 5 des Aufsatzes sicher und bequem mit dem weiblichen "ENLock"-Konnektor 10 der Überleiteinheit 6 verbunden werden, um so einen hermetisch versiegelten Nahrungsfluss aus dem Nahrungsbehälter 4 durch Spike 19 des Aufsatzes 1, weiter über die "ENLock"-Verbindung 5,10 in das Überleitsystem bereitzustellen.

## Patentansprüche

1. Vorrichtung zur enteralen Ernährung, umfassend
einen Nahrungsbehälter (4) mit daran angebrachtem Verbindungsstutzen (3),
eine Überleiteinheit (6), welche einen proximalen Konnektor (10) ohne Spike aufweist, und
einen Aufsatz (1), umfassend zumindest zwei Verbindungsstücke (2,5), wobei das erste Verbindungsstück (2) mit dem Verbindungsstutzen (3) des Nahrungsbehälters (4) verbunden ist und das zweite Verbindungsstück (5) mittels des proximalen Konnektors (10) mit der Überleiteinheit (6) verbunden ist, sodass ein Nahrungsfluss vom Nahrungsbehälter (4) durch den Verbindungsstutzen und den Aufsatz (1) zur Überleiteinheit (6) ermöglicht wird,
wobei der Aufsatz ein Mittel zum Öffnen (7) des Nahrungsbehälters (4) umfasst,
**dadurch gekennzeichnet, dass**
der Aufsatz (1) und der Verbindungsstutzen (3) irreversibel miteinander verbunden sind.

2. Vorrichtung nach Anspruch 1, wobei die Überleiteinheit (6) und der Aufsatz (1) formschlüssig über das zweite Verbindungsstück (5) des Aufsatzes (1) miteinander verbunden sind.

3. Vorrichtung nach Anspruch 1 oder 2, wobei das zweite Verbindungsstück (5) ein Arretierungselement aufweist.

4. Vorrichtung nach einem der vorhergehenden Ansprüche, wobei das zweite Verbindungsstück (5) als ENLock-Konnektor ausgebildet ist.

5. Vorrichtung nach einem der vorhergehenden Ansprüche, wobei das Mittel zum Öffnen (7) des Nahrungsbehälters (4) als Spike ausgeführt ist.

6. Vorrichtung nach einem der vorhergehenden Ansprüche, wobei das Mittel zum Öffnen (7) in Richtung des ersten Verbindungsstücks (2) bewegbar ist.

## Claims

1. Device for enteral nutrition, comprising
a food container (4), with a connection nozzle (3) mounted thereon,
a transfer unit (6), which has a proximal connector (10) without a spike, and
an attachment (1) comprising at least two connection pieces (2, 5), wherein the first connection piece (2) is connected to the connection nozzle (3) of the food container (4), and the second connection piece (5) is connected to the transfer unit (6) by means of the proximal connector (10), such that a flow of food is permitted from the food container (4) to the transfer unit (6) by way of the connection nozzle and the attachment (1),
wherein the attachment comprises a means (7) for opening the food container (4),
**characterized in that**
the attachment (1) and the connection nozzle (3) are connected to each other irreversibly.

2. Device according to Claim 1, wherein the transfer unit (6) and the attachment (1) are connected to each other with a form fit via the second connection piece (5) of the attachment (1).

3. Device according to Claim 1 or 2, wherein the second connection piece (5) has a locking element.

4. Device according to one of the preceding claims, wherein the second connection piece (5) is designed as an ENLock connector.

5. Device according to one of the preceding claims, wherein the means (7) for opening the food container (4) is designed as a spike.

6. Device according to one of the preceding claims, wherein the opening means (7) is movable in the direction of the first connection piece (2).

## Revendications

1. Dispositif pour la nutrition entérale, comprenant
un récipient d'aliments (4) auquel est raccordée une tubulure de liaison (3),
une unité de transfert (6) qui présente un connecteur proximal (10) sans pointe, et
un embout (1) comprenant au moins deux éléments de liaison (2, 5), le premier élément de liaison (2) étant relié à la tubulure de liaison (3) du récipient d'aliments (4) et le deuxième élément de liaison (5) étant relié au moyen du connecteur proximal (10) à l'unité de transfert (6), de telle sorte qu'un flux d'aliments puisse avoir lieu depuis le récipient d'aliments (4) à travers la tubulure de liaison et l'embout (1) jusqu'à l'unité de transfert (6),
l'embout comprenant un moyen pour ouvrir (7) le récipient d'aliments (4),
**caractérisé en ce que**
l'embout (1) et la tubulure de liaison (3) sont reliés l'un à l'autre de manière permanente.

2. Dispositif selon la revendication 1, dans lequel l'unité de transfert (6) et l'embout (1) sont reliés l'un à l'autre par engagement positif par le biais du deuxième élément de liaison (5) de l'embout (1).

3. Dispositif selon la revendication 1 ou 2, dans lequel le deuxième élément de liaison (5) présente un élément de blocage.

4. Dispositif selon l'une quelconque des revendications précédentes, dans lequel le deuxième élément de liaison (5) est réalisé sous forme de connecteur ENLock.

5. Dispositif selon l'une quelconque des revendications précédentes, dans lequel le moyen pour ouvrir (7) le récipient d'aliments (4) est réalisé sous forme de pointe.

6. Dispositif selon l'une quelconque des revendications précédentes, dans lequel le moyen pour ouvrir (7) peut être déplacé dans la direction du premier élément de liaison (2).
